# EUROPÄISCHE PATENTSCHRIFT

(11) **EP 2 571 845 B1**
(45) Veröffentlichungstag und Bekanntmachung des Hinweises auf die Patenterteilung: **01.10.2014**
(21) Anmeldenummer: 11720467.7
(22) Anmeldetag: 18.05.2011
(51) Int. Cl.: C07C 213/02, C07C 217/08

(54) **VERFAHREN ZUR HERSTELLUNG VON 2-(2-TERT.-BUTYLAMINO-ETHOXY)-ETHANOL (TERT.-BUTYLAMINODIGLYKOL, TBADG)**
METHOD FOR PRODUCING 2-(2-TERT.-BUTYLAMINO-ETHOXY)-ETHANOL (TERT.-BUTYLAMINODIGLYCOL, TBADG)
PROCÉDÉ DE PRÉPARATION DE 2-(2-TERT.-BUTYLAMINO-ÉTHOXY)-ÉTHANOL (TERT.-BUTYLAMINODIGLYCOL, TBADG)

(30) Priorität: 28.10.2010 EP 10189221; 21.05.2010 EP 10163583
(43) Veröffentlichungstag der Anmeldung: 27.03.2013
(73) Patentinhaber: BASF SE, 67056 Ludwigshafen (DE)
(72) Erfinder: BOU CHEDID, Roland, 68159 Mannheim (DE); MELDER, Johann-Peter, 67459 Böhl-Iggelheim (DE); BRUGHMANS, Steven, 68167 Mannheim (DE); KATZ, Torsten, 67434 Neustadt (DE)
(86) Internationale Anmeldenummer: PCT/EP2011/058030
(87) Internationale Veröffentlichungsnummer: WO 2011/144651

(56) Entgegenhaltungen:
- WO-A1-2010/031719
- US-A- 4 487 967

## Beschreibung

Die vorliegende Erfindung betrifft ein Verfahren zur Herstellung von 2-(2-tert.-Butylaminoethoxy)-ethanol (tert.-Butylaminodiglykol, TBADG) durch Umsetzung von Diethylenglykol (DG) mit tert.-Butylamin (TBA) in Gegenwart von Wasserstoff und eines kupferhaltigen Katalysators.

Das Verfahrensprodukt findet u.a. Verwendung in der Gaswäsche z. B. für die selektive Trennung von sauren Gasen wie z. B. H₂S aus Gasströmen die Mischungen von einem oder mehreren sauren Gasen und CO₂ beinhalten.

EP 137 478 A2 (BASF AG) betrifft ein Verfahren zur Herstellung von N-Methylpiperidin oder N-Methylmorpholin durch katalytische Aminierung von Pentandiol bzw. Diethylenglykol mit Methylamin in der Gasphase an einem kupferhaltigen Katalysator, der durch Temperung eines basischen Kupfer und Aluminium enthaltenden Carbonats erhalten wurde.

EP 235 651 A1 (BASF AG) lehrt ein Verfahren zur Herstellung von N-Methylpiperazin aus Diethanolamin und Methylamin an metallhaltigen Katalysatoren. Die Umsetzung wird in der Flüssigphase (Rieselfahrweise) durchgeführt (Seite 3, letzter Absatz). Gemäß Beispiel wird ein Cu/Al₂O₃-Katalysator eingesetzt.

EP 816 350 A1 (BASF AG) beschreibt Verfahren zur Herstellung von N-Methylpiperidin und N-Methylmorpholin durch Umsetzung von primärem Amin mit einem Diol an einem Kupferkatalysator, welcher durch Tränkung von SiO₂-Kugeln mit basischem Kupfercarbonat erhalten wurde, in der Flüssig- oder Gasphase.

US 4,739,051 A (BASF AG) lehrt die Herstellung von Morpholin und Piperidin durch Reaktion von DEG oder Pentandiol mit Ammoniak unter Hydrierbedingungen in der Gasphase bei Normaldruck und 200°C an einem Cu/NilAl-Vollkatalysator mit Ausbeuten von 97 bzw. 95 %.

EP 514 692 A2 (BASF AG) offenbart Verfahren zur Herstellung von Aminen aus Alkoholen in Gegenwart Kupfer und Nickel und Zirkonium- und/oder Aluminiumoxid enthaltender Katalysatoren.

DE 198 59 776 A1 (BASF AG) betrifft die Herstellung von Aminen durch Umsetzung von Alkoholen oder Aldehyden bzw. Ketonen mit Aminen an einem Katalysator aus Kupfer und TiO₂, welchem vor der Verformung des Katalysatormaterials metallisches Kupfer zugesetzt wurde. EP 440 829 A1 (US 4,910,304) (BASF AG) beschreibt die Aminierung von Diolen an Kupferkatalysatoren, insb. die Herstellung von N-Methylpiperidin und N-Methylmorpholin durch Reaktion von Pentandiol bzw. Diethylenglykol (DEG) mit Methylamin und 45 %iger wässriger KOH-Lösung an einem Cu/Al-Vollkontakt bei 245 °C und 250 bar. Die Umsetzung wird in der Flüssigphase (Rieselfahrweise) durchgeführt (Seite 3, letzter Absatz). Geeignete Katalysatoren sind die in DE 24 45 303 A (BASF AG) offenbarten Katalysatoren, die durch Temperung eines basischen Kupfer und Aluminium enthaltenen Carbonats der allgemeinen Zusammensetzung CuₘAl₆(CO₃)_{0,5m}O₃(OH)ₘ₋₁₂, wobei m einen beliebigen, auch nicht ganzzahligen, Wert zwischen 2 und 6 bedeutet, erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird.

WO 05/110969 A1 (BASF AG) beschreibt ein Verfahren zur kontinuierlichen Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300°C in Gegenwart eines kupferhaltigen Katalysators, wobei die katalytisch aktive Masse des Katalysators vor dessen Reduktion mit Wasserstoff 20 bis 85 Gew.-% Aluminiumoxid (Al₂O₃), Zirkoniumdioxid (ZrO₂), Titandioxid (TiO₂) und/oder Siliziumdioxid (SiO₂) enthält und die Umsetzung in der Gasphase isotherm in einem Rohrreaktor erfolgt.

WO 2010/031719 A1 (BASF SE) betrifft ein Verfahren zur kontinuierlichen Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300 °C in Gegenwart eines kupfer- und aluminiumoxidhaltigen Katalysators, wobei die Umsetzung in der Gasphase erfolgt und die katalytisch aktive Masse des Katalysators vor dessen Reduktion mit Wasserstoff Aluminiumoxid und sauerstoffhaltige Verbindungen des Kupfers enthält und der Katalysatorformkörper spezifiziert ist.

US 4,487,967 und US 4,665,195 (beide Exxon Res. & Eng. Co.) lehren die Herstellung von sterisch gehinderten Aminoetheralkoholen durch Umsetzung von entsprechenden Aminen mit Diethylenglykol bzw. Polyalkenyletherglykolen. Das Selektivitätsproblem bei der Umsetzung von TBA mit DEG aufgrund der Bildung von N-tert.-Butyl-morpholin (TBM) wird dargestellt (US 4,487,967: Spalte 3). Als Katalysatoren werden geträgerte und ungeträgerte Metalle, unter anderem Ni/Al₂O₃/SiO₂ - , Ni-Al - , Raney-Ni -, Raney-Cu - Katalysatoren eingesetzt. Beim genannten Cu-Katalysator liegt die TBADG-Ausbeute nur bei 6,4 % (US 4,487,967, Spalte 6, Tabelle 1). Beim Ni/Al₂O₃/SiO₂ - Katalysator beträgt die isolierte TBADG-Ausbeute nur 54 % (US 4,487,967, Spalte 5, Beispiel 1).

WO 07/021462 A2 (Exxon-Mobil Res. & Eng. Comp.) betrifft die Verwendung von Dialkylaminglykolen oder Monoalkylaminglykolethern in der Sauergaswäsche und ihre Herstellung durch Aminierung entsprechender Glykole. Seite 15 erwähnt die Herstellung von TBADG aus DEG und TBA an einem Ni-Katalysator in nur 30 % Ausbeute und geht dabei auch auf das Selektivitätsproblem ein (vgl. das Schema auf Seite 15).

WO 05/081778 A2 (Exxon-Mobil Res. & Eng. Comp.) beschreibt u.a. die Synthese von TBADG aus DEG und TBA an geträgerten Metall-Katalysatoren, wobei der Träger spezifische Porengrößen, Porenverteilungen und Oberflächen (BET) aufweist. Bevorzugt werden Ni-Katalysatoren (Seite 3, Absatz [0009]). In allen Beispielen werden trägerlose Ni-Katalysatoren verwendet. Beispielhafte Ergebnisse sind:
Example 6c, Seite 28, Run 27: DEG-Umsatz = 72 %, molares TBADG:TBM-Verhältnis = 13,
Example 9, Seite 31, 8 h: DEG-Umsatz = 62,5 %, molares TBADG:TBM-Verhältnis = 15, und
Example 12, Seite 37, #170-8: DEG-Umsatz = 51,9 %, TBADG:TBM-Masseverhältnis = 15,8 also molares TBADG:TBM-Verhältnis = 14.
Da hierbei keine Selektivitäten angegeben werden, kann keine Ausbeute errechnet werden. Bei einer TBADG-Selektivität von 80 % (bez. auf DEG) läge die TBADG-Ausbeute im besten Beispiel (Example 6c, Seite 28, Run 27) bei ca. 57 %.

US 4,405,585 (Exxon Res. & Eng. Comp.) beschreibt die Anwendung von stark sterisch gehinderten sekundären Aminoetheralkoholen zur selektiven Abtrennung von H₂S aus einem Gas beinhaltend CO₂ und H₂S. Beispiel 1 in Spalte 9 beschreibt die Herstellung von TBADG aus TBA und 2-Chlor-ethoxyethanol.

WO 05/082834 (Exxon-Mobil Res. & Eng. Comp.) beschreibt ein Verfahren zur Herstellung von sterisch stark gehinderten Aminoetheralkoholen und Diaminopolyalkenylethern durch Umsetzung eines primären Amins mit Polyalkylenglykol bei erhöhter Temperatur und Druck in Anwesenheit eines spezifischen Katalysators. Der Katalysator ist dadurch charakterisiert, dass bei seiner Herstellung organische Metallkomplexe auf einem Träger zersetzt werden.

Es wurde erfindungsgemäß erkannt, dass die Umsetzung von DG mit TBA an NickelKatalysatoren den sicherheitstechnisch erheblichen Nachteil hat, dass Zersetzungsprodukte von DG entstehen, die z. B. bei einer Betriebstörung des Reaktors (insb. Energieausfall), eine kritische Situation hervorrufen.
Bei der Aminierung von DG kommt es beispielsweise durch Decarbonylierung verstärkt zur Bildung von unerwünschten Komponenten wie Methoxyethanol, Methoxyethylamin, Methanol, Methan (siehe Schema unten). Das Methoxyethanol ist giftig, kann aufgrund seiner physikalischen Eigenschaften aus TBADG nur schlecht abgetrennt werden und kann damit zu Problemen hinsichtlich Spezifikation und Produktqualität führen.

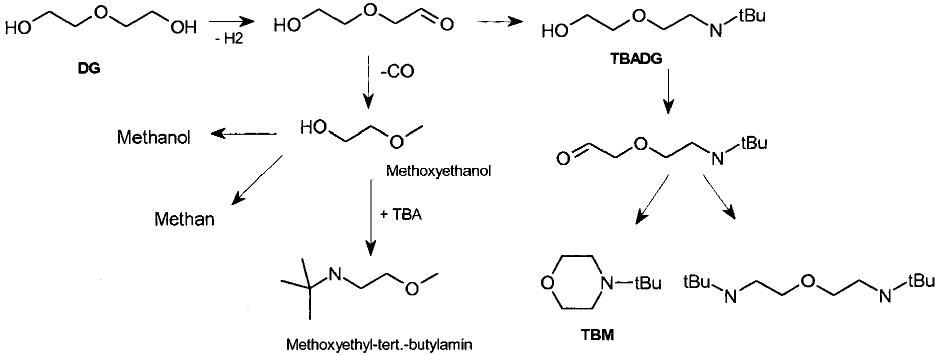

Um dieses Problem zu lösen, werden u.a. aufwendige Spezialreaktoren eingesetzt, vgl. z. B. WO 2009/092724 A1 (BASF SE), insb. Seite 10, Zeilen 14-21.

Der vorliegenden Erfindung lag die Aufgabe zugrunde, Nachteilen des Stands der Technik abzuhelfen und ein verbessertes wirtschaftliches Verfahren zur Herstellung von TBADG aufzufinden. Insbesondere soll das Verfahren zum einen hohe Ausbeuten, Raum-Zeit-Ausbeuten (RZA) und Selektivitäten ermöglichen und zum Anderen eine Decarbonylierung von DG und die damit verbundenen Nachteile unterdrücken und somit eine sichere Verfahrensführung ermöglichen. [Raum-Zeit-Ausbeuten werden angegeben in ,Produktmenge / (Katalysatorvolumen • Zeit)' (kg/(I_{Kat.} • h)) und/oder ,Produktmenge / (Reaktorvolumen • Zeit)' (kg/(I_{Reaktor} • h)].

Demgemäß wurde ein Verfahren Herstellung von 2-(2-tert.-Butylamino-ethoxy)-ethanol (tert.-Butylaminodiglykol, TBADG) durch Umsetzung von Diethylenglykol (DG) mit tert.-Butylamin (TBA) in Gegenwart von Wasserstoff und eines kupferhaltigen Katalysators gefunden, welches dadurch gekennzeichnet ist, dass die Umsetzung bei einer Temperatur im Bereich von 160 bis 220 °C in Gegenwart eines kupfer- und aluminiumoxidhaltigen Katalysators erfolgt, wobei die katalytisch aktive Masse des Katalysators vor dessen Reduktion mit Wasserstoff 20 bis 75 Gew.-% Aluminiumoxid (Al₂O₃), 20 bis 75 Gew.-% sauerstoffhaltige Verbindungen des Kupfers, berechnet als CuO, und ≤ 5 Gew.-% sauerstoffhaltige Verbindungen des Nickels, berechnet als NiO, enthält.

Bevorzugt ist das Verfahren dadurch gekennzeichnet, dass die Umsetzung nur bis zu einem DG-Umsatz im Bereich von 20 bis 80 %, besonders einem DG-Umsatz im Bereich von 30 bis 70 %, geführt wird.

Bevorzugt erfolgt die Umsetzung nicht in rein flüssiger Phase, sondern in der Gasphase oder Gas/Flüssig-Mischphase. Besonders bevorzugt erfolgt die Umsetzung in der Gasphase.

Bei einer Umsetzung in der Gas/Flüssig-Mischphase werden Wasserstoff (H₂) und DG bevorzugt in einem Molverhältnis von Wasserstoff : DG = 5 bis 50, besonders Wasserstoff : DG = 5 bis 30, eingesetzt.

Bei einer Umsetzung in der Gasphase werden Wasserstoff (H₂) und DG bevorzugt in einem Molverhältnis von Wasserstoff : DG = 40 bis 220, besonders Wasserstoff : DG = 50 bis 120, eingesetzt.

Das Molverhältnis (MV) von Wasserstoff : DG kann über den Druck und/oder eine Verdünnung mit einem Inertgas, z. B. N₂ oder Ar, eingestellt werden.

Bevorzugt erfolgt die Umsetzung in Abwesenheit eines Lösungsmittels.
Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Aluminiumoxid (Al₂O₃) als zur katalytisch aktiven Masse gehörig gewertet.
Die Katalysatoren werden dergestalt eingesetzt, dass man die katalytisch aktive Masse nach Mahlung, Vermischung mit Formhilfsmitteln, Formung und Temperung als Katalysatorformkörper - nämlich als Tabletten - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und des o. g. Katalysatorträgermaterials definiert und enthält im wesentlichen die folgenden Bestandteile:
Aluminiumoxid (Al₂O₃) und sauerstoffhaltige Verbindungen des Kupfers und bevorzugt sauerstoffhaltige Verbindungen des Natriums.

Die Summe der o. g. Bestandteile der katalytisch aktiven Masse, berechnet als Al₂O₃, CuO und Na₂O, beträgt üblicherweise 70 bis 100 Gew.-%, bevorzugt 80 bis 100 Gew.-%, besonders bevorzugt 90 bis 100 Gew.-%, weiter bevorzugt 98 bis 100 Gew.-%, weiter bevorzugt ≥ 99 Gew.-%, ganz besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Ni bzw. NiO, Co bzw. CoO, Re bzw. Rheniumoxide, Mn bzw. MnO₂, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie K₂O; Alkalimetallcarbonate, wie Na₂CO₃; Erdalkalimetalloxide, wie CaO, SrO; Erdalkalimetallcarbonate, wie MgCO₃, CaCO₃ und BaCO₃; Boroxid (B₂O₃).

Die katalytisch aktive Masse der im erfindungsgemäßen Verfahren eingesetzten Katalysatoren enthält nach deren letzter Wärmebehandlung und vor dessen Reduktion mit Wasserstoff 20 bis 75 Gew.-%, bevorzugt 25 bis 65 Gew.-%, besonders bevorzugt 30 bis 55 Gew.-%, Aluminiumoxid (Al₂O₃) und
20 bis 75 Gew.-%, bevorzugt 30 bis 70 Gew.-%, besonders bevorzugt 40 bis 65 Gew.-%, ganz besonders bevorzugt 45 bis 60 Gew.-%, sauerstoffhaltige Verbindungen des Kupfers, berechnet als CuO,
0 bis 2 Gew.-%, bevorzugt 0,05 bis 1 Gew.-%, besonders bevorzugt 0,1 bis 0,5
Gew.-%, sauerstoffhaltige Verbindungen des Natriums, berechnet als Na₂O,
≤ 5 Gew.-%, z. B. 0,1 bis 4 Gew.-%, bevorzugt weniger als 1 Gew.-%, z. B. 0 bis 0,8 Gew.-%, sauerstoffhaltige Verbindungen des Nickels, berechnet als NiO.

Die katalytisch aktive Masse des Katalysators enthält vor dessen Reduktion mit Wasserstoff besonders bevorzugt weniger als 1 Gew.-%, z. B. 0 bis 0,5 Gew.-%, sauerstoffhaltige Verbindungen des Kobalts, berechnet als CoO.

Ganz besonders bevorzugt enthält die katalytisch aktive Masse des im erfindungsgemäßen Verfahren eingesetzten Katalysators kein Nickel, kein Kobalt und/oder kein Ruthenium, jeweils weder in metallischer (Oxidationsstufe 0) noch in einer ionischen, insb. oxidierten, Form.

Bei den sauerstoffhaltigen Verbindungen des Kupfers handelt es sich insbesondere um Kupfer-(I)-oxid und Kupfer-(II)-oxid, bevorzugt um Kupfer-(II)-oxid.

Ganz besonders bevorzugt enthält die katalytisch aktive Masse des im erfindungsgemäßen Verfahren eingesetzten Katalysators kein Zirkoniumdioxid (ZrO₂), Titandioxid (TiO₂) und/oder Siliziumdioxid (SiO₂).

In einer besonders bevorzugten Ausführungsform enthält die katalytisch aktive Masse der im erfindungsgemäßen Verfahren eingesetzten Katalysatoren keine weitere katalytisch aktive Komponente, weder in elementarer noch in ionischer Form.
In der besonders bevorzugten Ausführungsform ist die katalytisch aktive Masse nicht mit weiteren Metallen oder Metallverbindungen dotiert.
Bevorzugt sind jedoch aus der Metallgewinnung von Cu, ggf. Ni, herrührende übliche Begleit-Spurenelemente hiervon ausgenommen.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten Aluminium, Kupfer, ggf. Natrium mit Wasser und nachfolgendes Extrudieren und Tempern der so erhaltenen Masse erhältlich.

Die im erfindungsgemäßen Verfahren verwendeten Katalysatoren können auch durch Tränkung von Aluminiumoxid (Al₂O₃), das beispielsweise in Form von Pulver oder Tabletten-Formkörpern vorliegt, hergestellt werden.

Aluminiumoxid kann dabei in verschiedenen Modifikationen eingesetzt werden, bevorzugt sind α- (alpha), γ- (gamma) oder θ-Al₂O₃ (theta-Al₂O₃). Besonders bevorzugt wird γ-Al₂O₃ eingesetzt.

Die Herstellung von Formkörpern des Aluminiumoxids kann nach den üblichen Verfahren erfolgen.

Die Tränkung des Aluminiumoxids erfolgt ebenfalls nach den üblichen Verfahren, wie z. B. in EP 599 180 A, EP 673 918 A oder A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. kalziniert.

Die Tränkung kann nach der sogenannten "incipient wetness"-Methode erfolgen, bei der das anorganische Oxid (d. h. Aluminiumoxid) entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu kalzinieren. Die mehrstufige Tränkung ist vorteilhaft besonders dann anzuwenden, wenn das anorganische Oxid mit einer größeren Metallmenge beaufschlagt werden soll.

Zur Aufbringung mehrerer Metallkomponenten auf das anorganische Oxid kann die Tränkung gleichzeitig mit ggf. allen Metallsalzen oder in beliebiger Reihenfolge der ggf. einzelnen Metallsalze nacheinander erfolgen.

Bevorzugt werden zur Herstellung der im erfindungsgemäßen Verfahren verwendeten Katalysatoren Fällungsmethoden angewandt. So können sie beispielsweise durch eine gemeinsame Fällung der Komponenten aus einer wässrigen Salzlösung mittels Mineralbasen in Gegenwart einer Aufschlämmung einer schwerlöslichen, sauerstoffhaltigen Aluminium-Verbindung und anschließendes Waschen, Trocknen und Kalzinieren des erhaltenen Präzipitats erhalten werden. Als schwerlösliche, sauerstoffhaltige Aluminiumverbindung kann beispielsweise Aluminiumoxid Verwendung finden. Die Aufschlämmungen der schwerlöslichen Aluminiumverbindung kann durch Suspendieren feinkörniger Pulver dieser Verbindung in Wasser unter kräftigem Rühren hergestellt werden. Vorteilhaft werden diese Aufschlämmungen durch Ausfällen der schwerlöslichen Aluminiumverbindung aus wässrigen Aluminium-Salzlösungen mittels Mineralbasen erhalten.

Bevorzugt werden die im erfindungsgemäßen Verfahren verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze des eingesetzten Metalls/der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden wie üblich zu den erfindungsgemäß verwendetenen Katalysatoren weiterverarbeitet. Nach dem Waschen werden sie im bevorzugt bei 80 bis 200 °C, vorzugsweise bei 100 bis 150 °C, getrocknet und danach kalziniert. Die Kalzinierung wird bevorzugt bei Temperaturen zwischen 300 und 800 °C, vorzugsweise 400 bis 600 °C, insbesondere 450 bis 550 °C, ausgeführt.

Nach der Kalzinierung wird der Katalysator zweckmäßigerweise konditioniert, sei es, dass man ihn durch Vermahlen auf eine bestimmte Korngröße einstellt und/oder dass man ihn nach seiner Vermahlung mit Formhilfsmitteln wie Graphit oder Stearinsäure vermischt, mittels einer Presse zu den Formlingen, nämlich Tabletten, verpresst und tempert. Die Tempertemperaturen entsprechen dabei bevorzugt den Temperaturen bei der Kalzinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200°C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400°C in einer Wasserstoffatmosphäre behandelt. Bei dieser Vorreduktion wird ein Teil der in den Katalysatoren vorliegenden sauerstoffhaltigen Metallverbindung/en zu dem/den entsprechenden Metall/en reduziert, so dass diese gemeinsam mit den verschiedenartigen Sauerstoffverbindungen in der aktiven Form des Katalysators vorliegen.

Der Katalysator ist bevorzugt charakterisiert durch ein Mikroporenvolumen von < 0,5 cm³/g, besonders < 0,4 cm³/g, z. B. < 0,3 cm³/g, (gemessen nach DI N 66135-1).
(Mikroporen sind nach sind gemäß der IUPAC-Empfehlung aus 1984 als Poren mit Porenweiten unter 2 nm definiert: K.S.W. Sing et al., Pure & Appl. Chem. 57 (1985) 4, 603-619).

Weiterhin ist der Katalysator bevorzugt charakterisiert durch folgende Porengrößenverteilung, wenn normalisiert auf Poren mit einer Porenweite von > 0 bis ≤ 20 nm (gemessen nach DIN 66134 (für die Mesoporen, Porenweite ≥ 2 bis ≤ 20 nm) und DIN 66135-1 (für die Mikroporen)): ≤ 30 % der Poren besitzen eine Porenweite bis 5 nm und mehr als 70 % der Poren besitzen eine Porenweite von > 5 bis 20 nm.

Die Umsetzung gemäß erfindungsgemäßem Verfahren erfolgt bevorzugt in einem Rohrreaktor.

Die Umsetzung im Rohrreaktor gemäß dem erfindungsgemäßen Verfahren erfolgt ganz besonders bevorzugt in einer Kreisgasfahrweise.

Das Kreisgas besteht aus überwiegend Wasserstoff oder aus einer Mischung von Wasserstoff und einem inerten Gas (z. B. N₂) und dient der Verdampfung der Edukte und/oder als Reaktionspartner für die Aminierungsreaktion.

In der Kreisgasfahrweise werden die Ausgangsstoffe (DG, TBA) bevorzugt in einem Kreisgasstrom verdampft und gasförmig dem Reaktor zugeführt.
Die Edukte (DG, TBA) können auch als wässrige Lösungen verdampft und mit dem Kreisgasstrom auf das Katalysatorbett geleitet werden.

Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Die Kreisgasmenge liegt bevorzugt im Bereich von 40 bis 2500 m³ (bei Betriebsdruck) / [m³ Katalysator (Schüttvolumen) h], insbesondere im Bereich von 100 bis 2000 m³ (bei Betriebsdruck) / [m³ Katalysator (Schüttvolumen) • h].

Das Kreisgas in eine Gas/Flüssig-Mischphasen-Fahrweise enthält bevorzugt mindestens 10, besonders 50 bis 100, ganz besonders 80 bis 100, Vol.-% H₂.

Das Kreisgas in eine Gasphasen-Fahrweise enthält bevorzugt mindestens 10, besonders 20 bis 80, ganz besonders 30 bis 60, Vol.-% H₂.

Die bevorzugt isotherme Umsetzung gemäß dem erfindungsgemäßen Verfahren erfolgt bevorzugt mit einer Temperaturabweichung von maximal +/- 8 °C, besonders maximal +/- 5 °C, insbesondere maximal +/- 4 °C, ganz besonders maximal +/- 3 °C, z. B. maximal +/- 0 bis +/- 2 °C oder maximal +/- 0 bis +/- 1 °C.
Diese Temperaturabweichungen beziehen sich auf die jeweiligen Temperaturen im jeweiligen Katalysatorbett, und zwar beim Eintritt der Edukte in das Katalysatorbett und beim Austritt der Reaktionsmischung aus dem Katalysatorbett.
Dabei können mehrere Katalysatorbetten parallel oder in Reihe ge- oder verschaltet sein.
Sind mehrere Katalysatorbetten in Reihe geschaltet, beziehen sich die genannten Temperaturabweichungen bei der erfindungsgemäß bevorzugten isothermen Fahrweise auf die jeweilige Temperatur im Katalysatorbett, und zwar beim Eintritt der Edukte in das erste Katalysatorbett und beim Austritt der Reaktionsmischung aus dem letzten Katalysatorbett.

In einer bevorzugten Ausführungsform erfolgt die Temperierung des Reaktorrohrs von außen mit einem Wärmeträgerstrom, wobei es sich bei dem Wärmeträger z. B. um ein Öl, eine Salzschmelze oder eine andere wärmeübertragende Flüssigkeit handeln kann.

Die erfindungsgemäße Reaktionsführung hat gegenüber einer Synthese in der nur-flüssigen Phase und im Besonderen gegenüber einer nicht-isothermen Synthese u.a. den Vorteil besserer Ausbeuten und einer höheren Sicherheit bezogen auf Durchgehreaktionen.
Durch die, bevorzugt isotherme, Gasphasen oder Gas/Flüssig-Mischphasen-Fahrweise, bevorzugt Gasphasen-Fahrweise, ist das Potential einer Durchgehreaktion während der Synthese stark reduziert. Die vorhandene Masse im Reaktor, die für eine Durchgehreaktion zur Verfügung stünde, ist nur ein Bruchteil der Masse eines nur-Flüssigphasen-Verfahrens.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

TBA und DG werden bevorzugt in einem Molverhältnis von TBA : DG = 1 bis 4, besonders in einem Molverhältnis von TBA : DG = 1 bis 3, weiter besonders in einem Molverhältnis von TBA : DG = 1 bis 2, eingesetzt.

Das erfindungsgemäße Verfahren wird bevorzugt bei einem Absolutdruck im Bereich von 1 bis 200 bar, bevorzugt 2 bis 100 bar, besonders bevorzugt 3 bis 50 bar, durchgeführt.

Das erfindungsgemäße Verfahren wird bevorzugt bei einer Temperatur im Bereich von 165 bis 205 °C, besonders bevorzugt 170 bis 200 °C, weiter bevorzugt 175 bis 195 °C, durchgeführt.

Die Katalysatorbelastung liegt bevorzugt im Bereich von 0,1 bis 2,0, bevorzugt 0,1 bis 1,0, besonders bevorzugt 0,2 bis 0,7 kg DG pro Liter Katalysator (Schüttvolumen) und Stunde. Die Anwendung höherer Katalysatorbelastungen ist möglich.

Der Druck im Reaktor, welcher sich aus der Summe der Partialdrücke des TBAs, DGs und der gebildeten Reaktionsprodukte bei den angegebenen Temperaturen ergibt, wird zweckmäßigerweise durch Aufpressen von Wasserstoff auf den gewünschten Reaktionsdruck erhöht.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben.

Unumgesetzte Edukte (DG und/oder TBA) und auch gegebenenfalls anfallende geeignete Nebenprodukte werden besonders bevorzugt wieder in die TBADG-Synthese zurückgeführt. Nicht umgesetztes TBA kann z. B. in diskontinuierlicher oder kontinuierlicher Fahrweise nach Kondensation der Produkte im Abscheider in dem Kreisgasstrom erneut über das Katalysatorbett geströmt werden. Nicht umgesetzte Edukte können auch nach einem oder mehreren, kontinuierlichen oder diskontinuierlichen Aufarbeitungsschritt/en, z.B. Destillation/en, in reiner Form oder gegebenenfalls auch als Mischung mit einer geeigneten Nebenkomponente in die Synthese zurückgeführt werden.

Alle Druckangaben beziehen sich auf den Absolutdruck.

### Beispiele

Eine Reihe von Versuchen zur Herstellung von 2-(2-tert.-Butylamino-ethoxy)-ethanol (TBADG) aus tert.-Butylamin (TBA) und Diethylenglykol (DG) in Gegenwart von Wasserstoff in diskontinuierlichen und kontinuierlichen Fahrweisen wurde durchgeführt. Es wurden Bedingungen gewählt, um die Umsätze und Selektivitäten in der Flüssigphase sowie in der Gasphase zu untersuchen. Der Katalysator wurde jeweils zunächst aktiviert und dann eingesetzt. Die Analytik wurde mittels Gaschromatographie (GC) auf einer Rtx-5-Amin Säule (mit 30 m Länge, 0,32 mm Innendurchmesser, 1,5 µm Beschichtung) und mit einem Temperaturprogramm von 60 °C bis 280 °C in 4 °C/min durchgeführt. Die quantitative Analyse erfolgte durch Bestimmung von Faktoren fürs DG, TBADG, N-tert.-Butylmorpholin (TBM) und das 2,2'-Di-(tert.-butylamino)-diethylether (DAE) mit Diethylenglykoldimethylether (DGDME) als Standard. Aus technischen Gründen konnte das TBA nicht quantitativ analysiert werden (Entspannungsverluste). Der Umsatz wurde nur bezogen auf das DG gerechnet.
Die Ergebnisse der Versuche sind in die Tabellen als Diethylenglykol Umsatz (Umsatz DG) in mol% vom eingesetzten DG, als TBADG Selektivität (Selektivität TBADG) in mol% vom umgesetztes DG, als Molverhältnis TBADG zum Nebenprodukt TBM (TBADG/TBM molar) und als TBADG Ausbeute (gerechnet aus Umsatz DG und Selektivität TBADG) eingegeben.

### Herstellung des nickelfreien Katalysators A (A1 und A2)

Der nickelfreie Kupfer-Katalysator A besaß die Zusammensetzung 50 Gew.-% CuO und 50 Gew.-% gamma-Al₂O₃ (nach dessen letzter Wärmebehandlung und vor dessen Reduktion mit Wasserstoff). Die Herstellung des Katalysators A erfolgte durch eine gemeinsame Fällung (Mischfällung) von Kupfer- und Aluminiumoxiden aus ihrer Nitratlösung (gemäß DE 30 27 890 A1, Seite 14 ff., Beispiel 1 und 2), Katalysator A1, oder durch Tränkung von gamma-Al₂O₃ - Pulver mit einer wässrigen Kupfernitrat-Lösung, Katalysator A2. Die Tablettierung erfolgte jeweils nach üblicher Methode. Vor Beginn der Umsetzung wurde der Katalysator im Wasserstoffstrom reduziert (s.u.).
Mikroporenvolumen der so erhaltenen Katalysatoren A1 und A2: 0,03 cm³/g. Porengrößenverteilung in den so erhaltenen Katalysatoren A1 und A2: Normalisiert auf Poren mit einer Porenweite von > 0 bis ≤ 20 nm (gemessen nach DIN 66134 (für die Mesoporen, Porenweite ≥ 2 bis ≤ 20 nm) und DIN 66135-1 (für die Mikroporen)) wiesen etwa 18 % der Poren eine Porenweite von kleiner als 5 nm auf.

### Vergleichsbeispiel 2 (VB-2) (diskontinuierliches Verfahren in der Mischphase)

DG (36,3 g, 0,34 mol), TBA (100,0 g, 1,36 mol), und Katalysator (5 g, aktivierter Vergleichskatalysator VK1, 28 Gew.-% Ni als NiO, 13 Gew.-% Cu als CuO auf Zirkoniumdioxid) wurden im Autoklaven (300 ml) vorgelegt. Der Autoklav wurde mit Stickstoff inertisiert und mit 50 bar Wasserstoff aufgepresst. Nach Aufheizen auf 195 °C wurde der Druck mit Wasserstoff auf 100 bar eingestellt und bei der Druckabnahme während der Reaktion wird Wasserstoff auf 100 bar wieder nachgepresst. Proben wurden genommen und mittels GC analysiert. Das Ergebnis ist in Tabelle 1, VB-2 eingetragen.

### Beispiel 2 (B-2) (diskontinuierliches Verfahren in der Mischphase)

DG (32,6 g, 0,31 mol), und TBA (90,0 g, 1,23 mol) wurden in einem mit Katalysatorkorb vorgesehenen Autoklav (300 ml) vorgelegt. Der aktivierte Katalysator A1 (5 g) wurde in den Katalysatorkorb gefüllt und in den Autoklav gestellt. Der Autoklav wurde mit Stickstoff inertisiert und mit 50 bar Wasserstoff aufgepresst. Nach Aufheizen auf 205 °C wurde der Druck mit Wasserstoff auf 100 bar eingestellt und bei der Druckabnahme während der Reaktion wird Wasserstoff auf 100 bar wieder nachgepresst. Proben wurden genommen und mittels GC analysiert. Das Ergebnis ist in Tabelle 1, B-2 eingetragen.

### Vergleichsbeispiel 3 (VB-3) (kontinuierliches Verfahren in der Gasphase)

Für die kontinuierliche Herstellung von TBADG wurde aus TBA und DG in ein Molverhältnis von 2:1 eine Zulaufmischung vorbereitet.
Die Aminierung wurde in einem mit Öl beheizten Doppelmantelreaktor (D = 40 mm, L = 900 mm) aus Glas mit ca. 1000 ml Volumen durchgeführt. Der Reaktor war mit einem Glasüberströmer auf 0,2 bar Überdruck gesichert und wurde bei normalem Druck betrieben. Der Zulauf wurde mittels einer Zulaufpumpe zusammen mit dem beheizten Wasserstoff am Kopf des senkrecht stehenden Reaktors zudosiert. Durch die Temperatur und die genügende Wasserstoff Menge wurde die Zulaufmischung am Kopf des Reaktors verdampft und im Reaktor gasförmig über den Katalysator geführt. Am Reaktorausgang unten befand sich eine Vorlage mit Intensivkühler wo die Ausgangsstoffe und die Produkte kondensiert und gesammelt wurden. Der Reaktor wurde mit ca. 200 ml Katalysator und drüber ca. 800 ml Metallringen aus V2A befüllt. Der Katalysator enthielt 46 Gew.-% Cu als CuO und 11 Gew.-% Ni als NiO auf Alox und wurde vor Beginn des Versuches bei 180-200 °C zuerst mit einem Wasserstoff/Stickstoff-Gemisch und später mit reinem Wasserstoff reduziert (Aktivierung). Dem Reaktor wurden dann bei 190 °C, 150 ml/h des Zulaufsgemisches (umgerechnet 54,2 g/h DG) und 160 Nl/h Wasserstoff von oben nach unten zugefahren. Das Ergebnis des Versuchs ist in Tabelle 2, VB-3 eingetragen. [Normliter (NI) = auf Normalbedingungen (20 °C, 1 bar) umgerechnetes Volumen].

### Beispiel 5 (B-5) (kontinuierliches Verfahren in der Gasphase)

150 ml/h einer Mischung aus TBA und DG in ein Molverhältnis von 2:1 und 160 Nl/h Wasserstoff wurde kontinuierlich bei normalem Druck (1 bar) und 190 °C auf 200 ml des Katalysators A1 in der gleichen Anlage wie in VB-3 gefahren. Der Katalysator wurde vor Beginn des Versuchs bei 180-200 °C zuerst mit einem Wasserstoff/Stickstoff-Gemisch und später mit reinem Wasserstoff reduziert (Aktivierung). Das Ergebnis ist in Tabelle 2, B-5 eingetragen.

### Beispiel 6 (B-6) (kontinuierliches Verfahren in der Gasphase)

150 ml/h einer Mischung aus TBA und DG in ein Molverhältnis von 3:1 und 160 Nl/h Wasserstoff wurde kontinuierlich bei normalem Druck (1 bar) und 190 °C auf 200 ml des Katalysators A1 in der gleichen Anlage wie in VB-3 gefahren. Der Katalysator wurde vor Beginn des Versuchs bei 180-200 °C zuerst mit einem Wasserstoff/Stickstoff-Gemisch und später mit reinem Wasserstoff reduziert (Aktivierung). Das Ergebnis ist in Tabelle 2, B-6 eingetragen.

### Beispiele 7, 8 und 10, 11 (B-7, B-8 und B-10, B-11)

Folgende Versuchsreihe (B-7 bis B-11) wurde in einem mit Öl beheizten Doppelmantelreaktor (D = 6 mm, L = 12,5 m) aus V2A mit ca. 350 ml Volumen durchgeführt. Der Zulauf und der Wasserstoff wurden am Kopf des senkrecht stehenden Reaktors kontinuierlich zudosiert.

### Beispiel 7 (B-7) (kontinuierliches Verfahren in der Mischphase)

260 ml/h einer Mischung aus TBA und DG in ein Molverhältnis von 4:1 und 1500 Nl/h Wasserstoff wurde kontinuierlich bei 200 bar und 180 °C auf 200 ml des Katalysators A1 gefahren. Der Katalysator wurde vor Anfang des Versuchs bei 180-200 °C zuerst mit einem Wasserstoff/Stickstoff-Gemisch und später mit reinem Wasserstoff reduziert (Aktivierung). Das Ergebnis ist in Tabelle 2, B-7 eingetragen.

### Beispiel 8 (B-8) (kontinuierlicher Verfahren in der Mischphase)

260 ml/h einer Mischung aus TBA und DG in ein Molverhältnis von 4:1 und 1500 Nl/h Wasserstoff wurde kontinuierlich bei 50 bar und 180 °C auf 200 ml des Katalysators A1 gefahren. Der Katalysator wurde vor Anfang des Versuchs bei 180-200 °C zuerst mit einem Wasserstoff/Stickstoff-Gemisch und später mit reinem Wasserstoff reduziert (Aktivierung). Das Ergebnis ist in Tabelle 2, B-8 eingetragen.

### Beispiel 10 (B-10) (kontinuierliches Verfahren in der Mischphase)

700 ml/h einer Mischung aus TBA und DG in ein Molverhältnis von 2:1 und 1500 Nl/h Wasserstoff wurde kontinuierlich bei 25 bar und 185 °C auf 200 ml des Katalysators A1 gefahren. Der Katalysator wurde vor Anfang des Versuchs bei 180-200 °C zuerst mit einem Wasserstoff/Stickstoff-Gemisch und später mit reinem Wasserstoff reduziert (Aktivierung). Das Ergebnis ist in Tabelle 2, B-10 eingetragen.

### Beispiel 11 (B-11) (kontinuierliches Verfahren in der Mischphase)

700 ml/h einer Mischung aus TBA und DG in ein Molverhältnis von 2:1 und 1500 Nl/h Wasserstoff wurde kontinuierlich bei 5 bar und 185 °C auf 200 ml des Katalysators A1 gefahren. Der Katalysator wurde vor Anfang des Versuchs bei 180-200 °C zuerst mit einem Wasserstoff/Stickstoff-Gemisch und später mit reinem Wasserstoff reduziert (Aktivierung). Das Ergebnis ist in Tabelle 2, B-11 eingetragen.

### Beispiele 12 bis 16 (B-12 bis B-16)

In folgender Versuchsreihe (B-12 bis B-16) wurde der Einfluss von Stickstoff zusammen mit Wasserstoff als Trägergas für die Umsetzung von DG mit TBA in der Gasphase untersucht. Die Aminierung wurde in einem mit Öl beheizten Doppelmantelreaktor (D = 41,1 mm, L = 3500 mm) aus V2A mit ca. 5 l Volumen durchgeführt. Der Zulauf wurde mittels einer Zulaufpumpe zusammen mit dem beheizten Wasserstoff am Kopf des senkrecht stehenden Reaktors zudosiert. Durch die eingestellte Temperatur und die genügende Wasserstoff-Menge wurde die Zulaufmischung am Kopf des Reaktors verdampft und im Reaktor gasförmig über den Katalysator geführt. Am Reaktorausgang befand sich ein Hochdruckabscheider bei einer Betriebstemperatur von 40 °C, in dem der Wasserstoff vom flüssigen Austrag abgetrennt wurde und mittels einer Pumpe in Kreis zurück in den Ofen gefahren wurde. Mit zwei zusätzlichen Pumpen wurde eine bestimmte Frischgasmenge Wasserstoff und eine bestimmte Menge Stickstoff zu dem Kreisgas zurückgefahren. Ein Druckregelventil regelte die Abgasmenge; damit wurde der Druck im System konstant gehalten. Der Reaktor wurde mit 1 Liter Katalysator A1 befüllt. Der Katalysator wurde vor Beginn des Versuches bei 180-200 °C zuerst mit einem Wasserstoff/StickstoffGemisch und später mit reinem Wasserstoff reduziert (Aktivierung).

### Beispiel 12 (B-12) (kontinuierliches Verfahren in der Gasphase)

3,1 l/h einer Mischung aus TBA und DG in einem Molverhältnis von 4:1 und 8,3 Nm³/h Wasserstoff wurde kontinuierlich bei 5 bar und 180 °C ohne Stickstoff gefahren. Das Verhältnis Wasserstoff zu Diethylenglykol lag bei 56:1 und die Verweilzeit bei ca. 2,5 Sekunden. Das Ergebnis ist in Tabelle 3, B-12 eingetragen.
[Normkubikmeter (Nm³) = auf Normalbedingungen (20 °C, 1 bar) umgerechnetes Volumen].

### Beispiel 13 (B-13) (kontinuierliches Verfahren in der Gasphase)

3,1 l/h einer Mischung aus TBA und DG in einem Molverhältnis von 4:1 und 15,3 Nm³/h Wasserstoff wurde kontinuierlich bei 10 bar und 180 °C ohne Stickstoff gefahren. Das Verhältnis Wasserstoff zu Diethylenglykol lag bei 112:1 und die Verweilzeit bei ca. 2,6 Sekunden. Das Ergebnis ist in Tabelle 3, B-13 eingetragen.

### Beispiel 14 (B-14) (kontinuierliches Verfahren in der Gasphase)

2,1 l/h einer Mischung aus TBA und DG in einem Molverhältnis von 4:1 und 19,3 Nm³/h Wasserstoff wurde kontinuierlich bei 20 bar und 180 °C ohne Stickstoff gefahren. Das Verhältnis Wasserstoff zu Diethylenglykol lag bei 217:1 und die Verweilzeit bei ca. 3,8 Sekunden. Das Ergebnis ist in Tabelle 3, B-14 eingetragen.

### Beispiel 15 (B-15) (kontinuierliches Verfahren in der Gasphase)

2,1 l/h einer Mischung aus TBA und DG in einem Molverhältnis von 4:1 und 19,3 Nm³/h einer Wasserstoff/Stickstoff Mischung von 1:1 wurde kontinuierlich bei 20 bar und 180 °C gefahren. Das Verhältnis Wasserstoff zu Diethylenglykol lag bei 107:1 und die Verweilzeit bei ca.
3,9 Sekunden. Das Ergebnis ist in Tabelle 3, B-15 eingetragen.

### Beispiel 16 (B-16) (kontinuierliches Verfahren in der Gasphase)

2,1 l/h einer Mischung aus TBA und DG in einem Molverhältnis von 4:1 und 19,3 Nm³/h einer Wasserstoff/Stickstoff Mischung von 1:1 wurde kontinuierlich bei 20 bar und 180 °C gefahren. Das Verhältnis Wasserstoff zu Diethylenglykol lag bei 71:1 und die Verweilzeit bei ca.
3,9 Sekunden. Das Ergebnis ist in Tabelle 3, B-16 eingetragen.

### Diskussion von Ergebnissen:

In Anwesenheit von einem Nickel-haltigen Kupfer-Katalysator, VB-2 (Tabelle 1), wurde die beste Ausbeute von 42 % TBADG nach 8 h erreicht, mit der besten Selektivität von 64 % bei einem Umsatz von 65 %. Die Selektivität am Anfang (4 h) ist gut und am Ende (12 h) geht sie zurück je höher der Umsatz wird.

In Anwesenheit von einem Nickel-freien Kupfer Katalysator, Beispiel B-2, wurde eine Ausbeute von 53 %, mit ein DG Umsatz von 71 % und eine Selektivität von 74 % nach 12 h erreicht. Die Selektivität von 74 % wurde auf dem Nickel-haltigen Katalysator in VB-2 nicht erreicht.
Ein Vorteil von Nickel-armen, insbesondere Nickel-freien Kupfer-Katalysatoren ist die Steigerung der Ausbeute um ca. 30 % durch die Verbesserung der TBADG-Selektivität durch Erhöhung des TBADG/TBM - Verhältnisses und durch Reduzierung der Zersetzung von DG.

Um den Vorteil eines Nickel-freien Kupfer-Katalysators gegenüber einem Nickel-haltigen Kupfer-Katalysator zu zeigen, wurden auch Versuche in der Gasphase bei 1 bar durchgeführt. In Vergleichsbeispiel VB-3 (Tabelle 2) wurde die Umsetzung auf einem Katalysator enthaltend 46 Gew.-% Cu als CuO und 11 Gew.-% Ni als NiO auf Alox durchgeführt und in Beispiel B-5 wurde die Umsetzung unter den gleichen Bedingungen auf dem Nickel-freien Kupfer Katalysator durchgeführt. Der Ni-haltige Katalysator produziert viel mehr vom unerwünschten TBM als der Ni-freie Katalysator. Selbst durch Reduzierung der Temperatur um 10 °C auf 180 °C (VB-4) bleibt das TBM als Hauptprodukt bestehen. Auf dem Nickel-freien Katalysator in B-5 ist das gewünschte Produkt TBADG das Hauptprodukt und wird mit einem Molverhältnis zu dem TBM von ca. 13,3 produziert. Das ist ein beachtlicher Vorteil bezüglich der Stoffkosten.
In den weiteren Versuchen B-7 bis B-11 in Tabelle 2 wurde der Einfluss des Drucks auf die Reaktion in einem kontinuierlichen Verfahren untersucht. Unter allen Bedingungen in diesen Versuchen befindet sich eine flüssige Phase und eine gasförmige Phase im Reaktor. Beim Reduzieren des Drucks von 200 bar auf 50 bar kann die Ausbeute von ca. 13 % auf ca. 32 % verbessert werden. Die Reduzierung des Drucks weiter auf 25 bar und 5 bar ermöglicht eine vergleichbare Ausbeute von ca. 30 % bei eine verdoppelte Belastung. Die Raumzeitausbeute kann also durch Reduzierung des Drucks von 50 bar auf 5 bar mehr als verdoppelt werden.

Die Reduzierung des Drucks hat zwei Effekte:
1) die Konzentration von Wasserstoff im System wird reduziert,
2) mehr DG und TBA befinden sich in der Gasphase.

Eine andere Reihe von Versuchen (B-12 bis B-16) wurde unter bestimmten Bedingungen durchgeführt, um eine einzige Gasphase (und keine Flüssigphase) im Reaktor zu halten. Die Kreisgas-Menge und ggf. die Belastung wurden angepasst, um die Bildung einer flüssige Phase bei höheren Druck zu vermeiden.
Die ersten drei Versuche zeigen wie der Umsatz von DG, die Selektivität und Ausbeute von TBADG sinken, wenn der Druck von 5 auf 10 und 20 bar erhöht wird. Das weist darauf hin, dass nicht nur der zweite o. g. Effekt ein Einfluss auf die Reaktion hat, sondern auch die Konzentration von Wasserstoff. Konkreter kann die Konzentration von Wasserstoff im System als das Molverhältnis Wasserstoff zu DG beschrieben werden. Das Molverhältnis (MV) H2 zu DG musste in den konkreten Beispielen bei höherem Druck von ca. 56 auf ca. 217 erhöht werden, um ein Gasphasen-System zu behalten.
Die Versuche B-15 und B-16 wurden unter den gleichen Bedingungen gefahren, außer, dass Wasserstoff mit Stickstoff stufenweise ersetzt worden ist. Die gesamte Kreisgasmenge wurde aber konstant gehalten, um die Bildung einer flüssigen Phase zu vermeiden. Das MV H2:DG wurde dadurch von 217 auf 107 und 71 reduziert. In Übereinstimmung mit den bisherige Beobachtungen wurden durch die Absenkung des MV H2:DG der Umsatz von DG und die Selektivität und die Ausbeute von TBADG verbessert.

**Tabelle 1**

| Versuch | Kat. | Temp. | Druck | MV TBA : DG | Probe x h | TBADG/TBM (molar) | DG Umsatz | TBADG Selektivität | TBADG Ausbeute |
|---|---|---|---|---|---|---|---|---|---|
| VB-2 | VK1 | 195 °C | 100 bar | 4:1 | 4 h | 4,0 | 49 % | 63 % | 31 % |
| | | | | | 8 h | 2,9 | 65 % | 64 % | 42 % |
| | | | | | 12 h | 1,3 | 85 % | 46 % | 39 % |
| B-2 | Cu/Alox | 195 °C | 100 bar | 4:1 | 12 h | 3,3 | 71 % | 74 % | 53 % |

**Tabelle 2**

| Versuch Nr. | Druck bar | Temp °C | Zulauf | | Bel. kg/l•h DG | H2 Nl/l•h | MV TBA:DG:H2 | Umsatz DG mol% | Selektivität TBADG mol% | Verhältnis TBADG/TBM (molar) | Ausbeute TBADG mol% |
|---|---|---|---|---|---|---|---|---|---|---|---|
| | | | g/h DG | ml/h Gesamt | | | | | | | |
| VB-3 | 1 | 190 | 54,2 | 150 | 0,27 | 800 | 2 : 1 : 14 | 83,2 | 13,3 | 0,2 | 11,1 |
| VB-4 | 1 | 180 | 54,2 | 150 | 0,27 | 800 | 2 : 1 :14 | 64,6 | 36,1 | 0,8 | 23,3 |
| | | | | | | | | | | | |
| B-5 | 1 | 190 | 54,2 | 150 | 0,27 | 800 | 2 : 1 : 14 | 67,8 | 70,0 | 13,3 | 47,4 |
| B-6 | 1 | 190 | 54,2 | 200 | 0,27 | 800 | 3 : 1 : 14 | 71,7 | 84,7 | 13,6 | 60,7 |
| | | | | | | | | | | | |
| B-7 | 200 | 180 | 50 | 260 | 0,27 | 7500 | 4 : 1 : 13 | 15,2 | 89,0 | 11,9 | 13,5 |
| B-8 | 50 | 180 | 50 | 260 | 0,26 | 7500 | 4 : 1 : 14 | 40,4 | 78,2 | 4,5 | 31,6 |
| B-10 | 25 | 185 | 130 | 700 | 0,62 | 7500 | 4 : 1 : 6 | 30,9 | 84,3 | 6,4 | 26,0 |
| B-11 | 5 | 185 | 130 | 700 | 0,62 | 7500 | 4 : 1 : 6 | 38,6 | 78,8 | 5,4 | 30,4 |

**Tabelle 3**

| Vers. Nr. | Druck (bar) | Temp. [°C] | Zulauf | | Bel. | Kreisgas [Nm³/h] | Frischgas [Nl/h] | MV TBA: DG | MV H2: DG | MV N2: H2 | Umsatz DG mol% | Selektivität TBADG mol% | Verhältnis TBADG/TBM molar | Ausbeute TBADG mol% |
|---|---|---|---|---|---|---|---|---|---|---|---|---|---|---|
| | | | kg/h DG | l/h ges. | (kg/l•h) DG | | | | | | | | | |
| B-12 | 5 | 180 | 636 | 3,1 | 0,64 | 8 | 300 | 4 | 56 | 0 | 30,0 | 64,8 | 1,9 | 19,4 |
| B-13 | 10 | 180 | 636 | 3,1 | 0,64 | 15 | 300 | 4 | 112 | 0 | 27,1 | 51,0 | 1,1 | 13,8 |
| B-14 | 20 | 180 | 424 | 2,1 | 0,42 | 19 | 300 | 4 | 217 | 0 | 19,7 | 46,0 | 0,9 | 9,0 |
| B-15 | 20 | 180 | 424 | 2,1 | 0,42 | 19 | 300 | 4 | 107 | 1 | 35,4 | 78,3 | 4,2 | 27,7 |
| B-16 | 20 | 180 | 424 | 2,1 | 0,42 | 19 | 300 | 4 | 71 | 2 | 30,3 | 83,4 | 6,2 | 25,2 |

## Patentansprüche

1. Verfahren zur Herstellung von 2-(2-tert.-Butylamino-ethoxy)-ethanol (tert.-Butyl-aminodiglykol, TBADG) durch Umsetzung von Diethylenglykol (DG) mit tert.-Butylamin (TBA) in Gegenwart von Wasserstoff und eines kupferhaltigen Katalysators, **dadurch gekennzeichnet, dass** die Umsetzung bei einer Temperatur im Bereich von 160 bis 220 °C in Gegenwart eines kupfer- und aluminiumoxidhaltigen Katalysators erfolgt, wobei die katalytisch aktive Masse des Katalysators vor dessen Reduktion mit Wasserstoff
20 bis 75 Gew.-% Aluminiumoxid (Al₂O₃),
20 bis 75 Gew.-% sauerstoffhaltige Verbindungen des Kupfers, berechnet als CuO, und
≤ 5 Gew.-% sauerstoffhaltige Verbindungen des Nickels, berechnet als NiO, enthält.

2. Verfahren nach dem vorhergehenden Anspruch, **dadurch gekennzeichnet, dass** die Umsetzung in der Gasphase oder Gas/Flüssig-Mischphase erfolgt.

3. Verfahren nach einem der beiden vorhergehenden Ansprüche, **dadurch gekennzeichnet, dass** die Umsetzung nur bis zu einem DG-Umsatz im Bereich von 20 bis 80 % geführt wird.

4. Verfahren nach dem vorhergehenden Anspruch, **dadurch gekennzeichnet, dass** unumgesetzes DG und/oder TBA wieder in die Umsetzung zurückgeführt wird.

5. Verfahren nach einem der vorhergehenden Ansprüche, **dadurch gekennzeichnet, dass** die Umsetzung bei einer Temperatur im Bereich von 170 bis 205 °C erfolgt.

6. Verfahren nach einem der vorhergehenden Ansprüche, **dadurch gekennzeichnet, dass** TBA und DG in einem Molverhältnis von TBA : DG = 1 bis 4 eingesetzt werden.

7. Verfahren nach einem der vorhergehenden Ansprüche 2 bis 6, **dadurch gekennzeichnet, dass** Wasserstoff (H₂) und DG in einem Molverhältnis von Wasserstoff : DG = 5 bis 50 bei einer Umsetzung in der Gas/Flüssig-Mischphase bzw. in einem Molverhältnis von Wasserstoff : DG = 40 bis 220 bei einer Umsetzung in der Gasphase eingesetzt werden.

8. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Nickels, berechnet als NiO, enthält.

9. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Kobalts, berechnet als CoO, enthält.

10. Verfahren nach einem der vorhergehenden Ansprüche, **dadurch gekennzeichnet, dass** die katalytisch aktive Masse des Katalysators vor dessen Reduktion mit Wasserstoff
25 bis 65 Gew.-% Aluminiumoxid (Al₂O₃) und
30 bis 70 Gew.-% sauerstoffhaltige Verbindungen des Kupfers, berechnet als CuO, enthält.

11. Verfahren nach einem der vorhergehenden Ansprüche, **dadurch gekennzeichnet, dass** die katalytisch aktive Masse des Katalysators vor dessen Reduktion mit Wasserstoff 0 bis 2 Gew.-% sauerstoffhaltige Verbindungen des Natriums, berechnet als Na₂O, enthält.

12. Verfahren nach einem der vorhergehenden Ansprüche, **dadurch gekennzeichnet, dass** die katalytisch aktive Masse des Katalysators vor dessen Reduktion mit Wasserstoff 0,05 bis 1 Gew.-% sauerstoffhaltige Verbindungen des Natriums, berechnet als Na₂O, enthält.

13. Verfahren nach einem der vorhergehenden Ansprüche, **dadurch gekennzeichnet, dass** die katalytisch aktive Masse des Katalysators kein Nickel, Kobalt und/oder Ruthenium enthält.

14. Verfahren nach einem der vorhergehenden Ansprüche, **dadurch gekennzeichnet, dass** die Umsetzung isotherm, mit einer Temperaturabweichung von maximal +/- 8°C erfolgt.

15. Verfahren nach einem der vorhergehenden Ansprüche, **dadurch gekennzeichnet, dass** die Umsetzung in Abwesenheit eines Lösungsmittels erfolgt.

16. Verfahren nach einem der vorhergehenden Ansprüche, **dadurch gekennzeichnet, dass** die Umsetzung kontinuierlich erfolgt.

17. Verfahren nach dem vorhergehenden Anspruch, **dadurch gekennzeichnet, dass** die Umsetzung in einem Rohrreaktor erfolgt.

18. Verfahren nach einem der beiden vorhergehenden Ansprüche, **dadurch gekennzeichnet, dass** die Umsetzung im Rohrreaktor bei einer Kreisgasfahrweise erfolgt.

19. Verfahren nach dem vorhergehenden Anspruch, **dadurch gekennzeichnet, dass** die Kreisgasmenge im Bereich von 40 bis 2500 m³ (bei Betriebsdruck) / [m³ Katalysator (Schüttvolumen) • h] liegt.

20. Verfahren nach einem der beiden vorhergehenden Ansprüche, **dadurch gekennzeichnet, dass** das Kreisgas mindestens 10 Vol.-% Wasserstoff (H₂) enthält.

21. Verfahren nach einem der vorhergehenden Ansprüche, **dadurch gekennzeichnet, dass** man die Umsetzung bei einem Absolutdruck im Bereich von 1 bis 200 bar durchführt.

22. Verfahren nach einem der vorhergehenden Ansprüche, **dadurch gekennzeichnet, dass** der Katalysator im Reaktor als Festbett angeordnet ist.

23. Verfahren nach einem der vorhergehenden Ansprüche, **dadurch gekennzeichnet, dass** die Umsetzung in Gegenwart eines Katalysators erfolgt, der ein Mikroporenvolumen von < 0,5 cm³/g aufweist.

24. Verfahren nach einem der vorhergehenden Ansprüche, **dadurch gekennzeichnet, dass** die Umsetzung in Gegenwart eines Katalysators erfolgt, in dem, normalisiert auf Poren mit einer Porenweite von > 0 bis ≤ 20 nm, ≤ 30 % der Poren eine Porenweite bis 5 nm und mehr als 70 % der Poren eine Porenweite von > 5 bis 20 nm aufweisen.

## Claims

1. A process for preparing 2-(2-tert-butylaminoethoxy)ethanol (tert-butylaminodiglycol, TBADG) by reacting diethylene glycol (DG) with tert-butylamine (TBA) in the presence of hydrogen and of a copper catalyst, which comprises effecting the reaction at a temperature in the range from 160 to 220°C in the presence of a copper- and aluminum oxide-containing catalyst, where the catalytically active material of the catalyst, before the reduction thereof with hydrogen, comprises
20 to 75% by weight of aluminum oxide (Al₂O₃),
20 to 75% by weight of oxygen compounds of copper, calculated as CuO, and
≤ 5% by weight of oxygen compounds of nickel, calculated as NiO.

2. The process according to the preceding claim, wherein the reaction is effected in the gas phase or gas/liquid mixed phase.

3. The process according to either of the two preceding claims, wherein the reaction is conducted only up to a DG conversion in the range from 20 to 80%.

4. The process according to the preceding claim, wherein unconverted DG and/or TBA is recycled back into the reaction.

5. The process according to any of the preceding claims, wherein the reaction is effected at a temperature in the range from 170 to 205°C.

6. The process according to any of the preceding claims, wherein TBA and DG are used in a molar ratio of TBA:DG = 1 to 4.

7. The process according to any of the preceding claims 2 to 6, wherein hydrogen (H₂) and DG are used in a molar ratio of hydrogen:DG = 5 to 50 in a reaction in the gas/liquid mixed phase, or in a molar ratio of hydrogen:DG = 40 to 220 in a reaction in the gas phase.

8. The process according to any of the preceding claims, wherein the catalytically active material of the catalyst, before the reduction thereof with hydrogen, comprises less than 1% by weight of oxygen compounds of nickel, calculated as NiO.

9. The process according to any of the preceding claims, wherein the catalytically active material of the catalyst, before the reduction thereof with hydrogen, comprises less than 1% by weight of oxygen compounds of cobalt, calculated as CoO.

10. The process according to any of the preceding claims, wherein the catalytically active material of the catalyst, before the reduction thereof with hydrogen, comprises
25 to 65% by weight of aluminum oxide (Al₂O₃) and
30 to 70% by weight of oxygen compounds of copper, calculated as CuO.

11. The process according to any of the preceding claims, wherein the catalytically active material of the catalyst, before the reduction thereof with hydrogen, comprises 0 to 2% by weight of oxygen compounds of sodium, calculated as Na₂O.

12. The process according to any of the preceding claims, wherein the catalytically active material of the catalyst, before the reduction thereof with hydrogen, comprises 0.05 to 1% by weight of oxygen compounds of sodium, calculated as Na₂O.

13. The process according to any of the preceding claims, wherein the catalytically active material of the catalyst does not comprise any nickel, cobalt and/or ruthenium.

14. The process according to any of the preceding claims, wherein the reaction is effected isothermally, with a temperature deviation of not more than +/- 8°C.

15. The process according to any of the preceding claims, wherein the reaction is effected in the absence of a solvent.

16. The process according to any of the preceding claims, wherein the reaction is effected continuously.

17. The process according to the preceding claim, wherein the reaction is effected in a tubular reactor.

18. The process according to either of the two preceding claims, wherein the reaction is effected in a tubular reactor in a cycle gas mode.

19. The process according to the preceding claim, wherein the cycle gas rate is in the range from 40 to 2500 m³ (at operating pressure)/[m³ of catalyst (bed volume) • h].

20. The process according to either of the two preceding claims, wherein the cycle gas comprises at least 10% by volume of hydrogen (H₂).

21. The process according to any of the preceding claims, wherein the reaction is performed at an absolute pressure in the range from 1 to 200 bar.

22. The process according to any of the preceding claims, wherein the catalyst is arranged as a fixed bed in the reactor.

23. The process according to any of the preceding claims, wherein the reaction is effected in the presence of a catalyst which has a micropore volume of < 0.5 cm³/g.

24. The process according to any of the preceding claims, wherein the reaction is effected in the presence of a catalyst in which, normalized to pores having a pore size of > 0 to 20 nm, ≤ 30% of the pores have a pore size up to 5 nm and more than 70% of the pores have a pore size of > 5 to 20 nm.

## Revendications

1. Procédé de fabrication de 2-(2-tert.-butylaminoéthoxy)-éthanol (tert.-butyl-aminodiglycol TBADG) par mise en réaction de diéthylène glycol (DG) avec de la tert.-butylamine (TBA) en présence d'hydrogène et d'un catalyseur contenant du cuivre, **caractérisé en ce que** la réaction a lieu à une température dans la plage allant de 160 à 220 °C en présence d'un catalyseur contenant de l'oxyde de cuivre et d'aluminium, la masse catalytiquement active du catalyseur contenant avant sa réduction avec de l'hydrogène
20 à 75 % en poids d'oxyde d'aluminium (Al₂O₃),
20 à 75 % en poids de composés oxygénés de cuivre, calculés en tant que CuO, et
≤ 5 % en poids de composés oxygénés de nickel, calculés en tant que NiO.

2. Procédé selon la revendication précédente, **caractérisé en ce que** la réaction a lieu en phase gazeuse ou en phase mixte gaz/liquide.

3. Procédé selon l'une quelconque des deux revendications précédentes, **caractérisé en ce que** la réaction n'est réalisée que jusqu'à une conversion du DG dans la plage allant de 20 à 80 %.

4. Procédé selon la revendication précédente, **caractérisé en ce que** le DG et/ou la TBA non réagis sont recyclés dans la réaction.

5. Procédé selon l'une quelconque des revendications précédentes, **caractérisé en ce que** la réaction a lieu à une température dans la plage allant de 170 à 205 °C.

6. Procédé selon l'une quelconque des revendications précédentes, **caractérisé en ce que** la TBA et le DG sont utilisés en un rapport en moles TBA:DG = 1 à 4.

7. Procédé selon l'une quelconque des revendications 2 à 6 précédentes, **caractérisé en ce que** l'hydrogène (H₂) et le DG sont utilisés en un rapport en moles hydrogène:DG = 5 à 50 lors d'une réaction en phase mixte gaz/liquide ou en un rapport en moles hydrogène:DG = 40 à 220 lors d'une réaction en phase gazeuse.

8. Procédé selon l'une quelconque des revendications précédentes, **caractérisé en ce que** la masse catalytiquement active du catalyseur contient avant sa réduction avec de l'hydrogène moins de 1 % en poids de composés oxygénés de nickel, calculés en tant que NiO.

9. Procédé selon l'une quelconque des revendications précédentes, **caractérisé en ce que** la masse catalytiquement active du catalyseur contient avant sa réduction avec de l'hydrogène moins de 1 % en poids de composés oxygénés de cobalt, calculés en tant que CoO.

10. Procédé selon l'une quelconque des revendications précédentes, **caractérisé en ce que** la masse catalytiquement active du catalyseur contient avant sa réduction avec de l'hydrogène
25 à 65 % en poids d'oxyde d'aluminium (Al₂O₃) et
30 à 70 % en poids de composés oxygénés de cuivre, calculés en tant que CuO.

11. Procédé selon l'une quelconque des revendications précédentes, **caractérisé en ce que** la masse catalytiquement active du catalyseur contient avant sa réduction avec de l'hydrogène 0 à 2 % en poids de composés oxygénés de sodium, calculés en tant que Na₂O.

12. Procédé selon l'une quelconque des revendications précédentes, **caractérisé en ce que** la masse catalytiquement active du catalyseur contient avant sa réduction avec de l'hydrogène 0,05 à 1 % en poids de composés oxygénés de sodium, calculés en tant que Na₂O.

13. Procédé selon l'une quelconque des revendications précédentes, **caractérisé en ce que** la masse catalytiquement active du catalyseur ne contient pas de nickel, de cobalt et/ou de ruthénium.

14. Procédé selon l'une quelconque des revendications précédentes, **caractérisé en ce que** la réaction a lieu de manière isotherme, avec une déviation de température d'au plus ±8 °C.

15. Procédé selon l'une quelconque des revendications précédentes, **caractérisé en ce que** la réaction a lieu en l'absence d'un solvant.

16. Procédé selon l'une quelconque des revendications précédentes, **caractérisé en ce que** la réaction a lieu en continu.

17. Procédé selon la revendication précédente, **caractérisé en ce que** la réaction a lieu dans un réacteur tubulaire.

18. Procédé selon l'une quelconque des deux revendications précédentes, **caractérisé en ce que** la réaction a lieu dans le réacteur tubulaire en un mode à gaz circulaire.

19. Procédé selon la revendication précédente, **caractérisé en ce que** la quantité de gaz circulaire se situe dans la plage allant de 40 à 2 500 m³ (à la pression d'exploitation)/[m³ de catalyseur (volume apparent)·h].

20. Procédé selon l'une quelconque des deux revendications précédentes, **caractérisé en ce que** le gaz circulaire contient au moins 10 % en volume d'hydrogène (H₂).

21. Procédé selon l'une quelconque des revendications précédentes, **caractérisé en ce que** la réaction est réalisée à une pression absolue dans la plage allant de 1 à 200 bar.

22. Procédé selon l'une quelconque des revendications précédentes, **caractérisé en ce que** le catalyseur est agencé dans le réacteur sous la forme d'un lit fixe.

23. Procédé selon l'une quelconque des revendications précédentes, **caractérisé en ce que** la réaction a lieu en présence d'un catalyseur qui présente un volume de micropores < 0,5 cm³/g.

24. Procédé selon l'une quelconque des revendications précédentes, **caractérisé en ce que** la réaction a lieu en présence d'un catalyseur dans lequel, normalisé par rapport aux pores ayant une largeur de pore de > 0 à ≤ 20 nm, ≤ 30 % des pores présentent une largeur de pore de jusqu'à 5 nm et plus de 70 % des pores présentent une largeur de pore de > 5 à 20 nm.
